# EUROPEAN PATENT SPECIFICATION

(11) **EP 4 091 529 B1**
(45) Date of publication and mention of the grant of the patent: **29.10.2025**
(21) Application number: 22174437.8
(22) Date of filing: 19.05.2022
(51) Int. Cl.: A61B 1/00, G16H 30/20, G16H 30/40

(54) **MEDICAL IMAGE PROCESSING SYSTEM AND METHOD FOR OPERATING THE SAME**
MEDIZINISCHES BILDVERARBEITUNGSSYSTEM UND VERFAHREN ZUM BETRIEB DESSELBEN
SYSTÈME DE TRAITEMENT D'IMAGES MÉDICALES ET SON PROCÉDÉ DE FONCTIONNEMENT

(30) Priority: 21.05.2021 JP 2021086546
(43) Date of publication of application: 23.11.2022
(73) Proprietor: FUJIFILM Corporation, Tokyo 106-8620 (JP)
(72) Inventor: TERAMURA, Yuichi, Kanagawa (JP)
(74) Representative: Dehns Germany Partnerschaft mbB

(56) References cited:
- US-A1- 2017 337 684
- CURTIS JAMES A ET AL: "Visual Analysis of Swallowing Efficiency and Safety (VASES): A Standardized Approach to Rating Pharyngeal Residue, Penetration, and Aspiration During FEES", DYSPHAGIA, SPRINGER, BOSTON, vol. 37, no. 2, 10 April 2021 (2021-04-10), pages 417 - 435, XP037750703, ISSN: 0179-051X, [retrieved on 20210410], DOI: 10.1007/S00455-021-10293-5

## Description

### BACKGROUND OF THE INVENTION

### 1. Field of the Invention

The present invention relates to a medical image processing system and a method for operating the same.

### 2. Description of the Related Art

In a case in which an endoscope is inserted into a subject for observation, a moving image may be captured in order to record a movement in the subject or to prevent a still image from being missed. An endoscope operator presses a recording button of a moving image recording device to start imaging before starting observation or inserting an endoscope tip portion into the subject and continues the imaging until the end of the observation or until the endoscope tip portion is removed from the subject. Therefore, the recording time of the acquired moving image file increases. A swallowing endoscope is used to acquire a series of swallowing aspects as a moving image. However, a portion used for diagnosis is often less than 5 seconds in one swallowing movement, and a plurality of swallowing reactions are observed during a single examination. Since the moving image recording time of recording one swallow movement is several minutes or more, a loss occurs in a case in which the target swallowing is checked later. Therefore, in a case in which swallowing is diagnosed, it is necessary to selectively play back only a swallowing portion of the acquired moving image file.

However, in the diagnosis of swallowing, it is inefficient to search for a target portion by designating the playback time or fast-forwarding the moving image, and the management of the moving image file tends to be complicated. Since the part to be imaged is almost the same, it is difficult to easily check the results of each moving image. In addition, in a case in which the playback time is long, it takes time to review the moving image.

WO2018/043585A discloses a technique which performs a freezing operation at any time to create a chapter image in a case in which an image is acquired with an endoscope and plays back the chapter image from a chapter image acquisition point after the end of observation or compares the chapter image with images captured in the past. JP2017-510368A discloses a technique which evaluates the characteristics of a swallowing process of a subject in a case in which the subject swallows food by associating a vibration sensor with an imaging technique. US 2017/337684 also teaches an imaging technique for examining swallowing function.

Aghdam et al. "A Comparison of Visual Recognition of the Laryngopharyngeal Structures Between High and Standard Frame Rate Videos of the Fiberoptic Endoscopic Evaluation of Swallowing." Dysphagia, vol. 32, no. 5, 2017, pp. 617-625 discloses how endoscopic high-speed videos improve structure recognition during swallowing compared to standard videos.

### SUMMARY OF THE INVENTION

In WO2018/043585A, since the chapter image is acquired by the operation of the user, for example, the missing of the image or the deviation of a playback position occurs. In addition, WO2018/043585A does not disclose the execution of the observation of swallowing or the pharynx. In JP2017-510368A, the movement of swallowing is sensed and the swallowing characteristics are evaluated using the imaging technique. However, this is a method that captures an image after sensing the vibration of swallowing, and JP2017-510368A does not disclose the execution of the observation of swallowing only with images. Based on the above points, it is desirable to minimize the observation time of swallowing by a moving image and to efficiently observe the swallowing by accurately performing the detection of the swallowing and the extraction of the images obtained by imaging the swallowing without a time lag.

An object of the invention is to provide a medical image processing system that can automatically extract an index moving image from a moving image obtained by imaging a swallowing examination and automatically play back the index moving image after the swallowing examination ends and a method for operating the same.

According to an aspect of the invention, there is provided a medical image processing system comprising a processor. The processor receives a video signal on which a swallowing examination has been recorded by an endoscope, analyzes the video signal to determine whether or not a swallowing timing is present, sets a frame image at the swallowing timing as a swallowing frame image tagged with swallowing timing detection, and extracts an index moving image including the swallowing frame image from the video signal.

Preferably, the index moving image includes a swallowing frame image group including the swallowing frame image and a frame image group for a predetermined period which is continuous with the swallowing frame image group.

Preferably, the frame image group for the predetermined period is non-swallowing frame images which are arranged before a start of the swallowing frame image group and after an end of the swallowing frame image group and are not tagged with the swallowing timing detection.

Preferably, the video signal includes a frame image to be analyzed, and the processor determines whether or not the swallowing timing is present using any one of calculation of an amount of blur of the frame image, calculation of a key point based on the frame image, or a difference between pixel values of the frame images.

Preferably, the processor analyzes the index moving image to specify a type of the swallowing examination.

Preferably, the processor gives an index number used to search for a moving image to the index moving image.

Preferably, the processor automatically plays back the index moving image without any user operation in a case in which the index moving image is displayed on a screen.

Preferably, the processor displays a plurality of the index moving images in a list on a display and automatically plays back the plurality of index moving images simultaneously or continuously.

Preferably, the processor displays at least one of a type of the swallowing examination or an index number in a case in which the index moving image is displayed on a screen.

Preferably, the processor combines a plurality of the index moving images to create a composite index moving image in which the index moving images are capable of being continuously played back.

Preferably, the processor determines whether or not the swallowing timing is present further using voice recognition at the time of swallowing.

According to another aspect of the invention, there is provided a method for operating a medical image processing system including a processor. The method comprises: a step of causing the processor to receive a video signal on which a swallowing examination has been recorded by an endoscope; a step of causing the processor to analyze the video signal to determine whether or not a swallowing timing is present and to set a frame image at the swallowing timing as a swallowing frame image tagged with swallowing timing detection; and a step of causing the processor to extract an index moving image including the swallowing frame image from the video signal.

It is possible to automatically extract an index moving image from a moving image obtained by imaging a swallowing examination and to automatically play back the index moving image after the swallowing examination ends. Therefore, the user can efficiently observe swallowing.

### BRIEF DESCRIPTION OF THE DRAWINGS

Fig. 1 is a schematic diagram illustrating a medical image processing system.
Fig. 2 is a diagram illustrating food swallowing stages.
Fig. 3 is a block diagram illustrating functions of a medical image processing device.
Fig. 4 is a diagram illustrating the detection of a swallowing timing from a video signal and the extraction of an index moving image.
Fig. 5 is an image diagram illustrating observation images of the pharynx in a case in which food is swallowed and in a case in which food is not swallowed.
Fig. 6 is an image diagram illustrating the extraction of a plurality of index moving images from the video signal.
Fig. 7 is an image diagram illustrating a classification result of swallowing and the distribution of the classification result to index numbers.
Fig. 8 is an image diagram illustrating a case in which a list of index moving images is displayed on a screen.
Fig. 9 is an image diagram illustrating the display of one index moving image on the screen.
Fig. 10 is an image diagram illustrating an index moving image search screen.
Fig. 11 is an image diagram illustrating the combination of the index moving images.
Fig. 12 is a diagram and an image diagram illustrating an imaging method in a swallowing examination.
Fig. 13 is a diagram illustrating a method for determining swallowing using a pixel value difference between front and rear frame images.
Fig. 14 is a diagram illustrating a method for determining swallowing using the blur of a frame image.
Fig. 15 is a diagram illustrating a method for determining swallowing using the number of key points in the frame image.
Fig. 16 is a flowchart illustrating the extraction of an index moving image from a video signal.
Fig. 17 is a block diagram illustrating functions of a swallowing timing detection unit implemented in a second embodiment.
Fig. 18 is an image diagram illustrating the simultaneous display of an extracted index moving image and a past index moving image which is implemented in a third embodiment.

### DESCRIPTION OF THE PREFERRED EMBODIMENTS

### First Embodiment

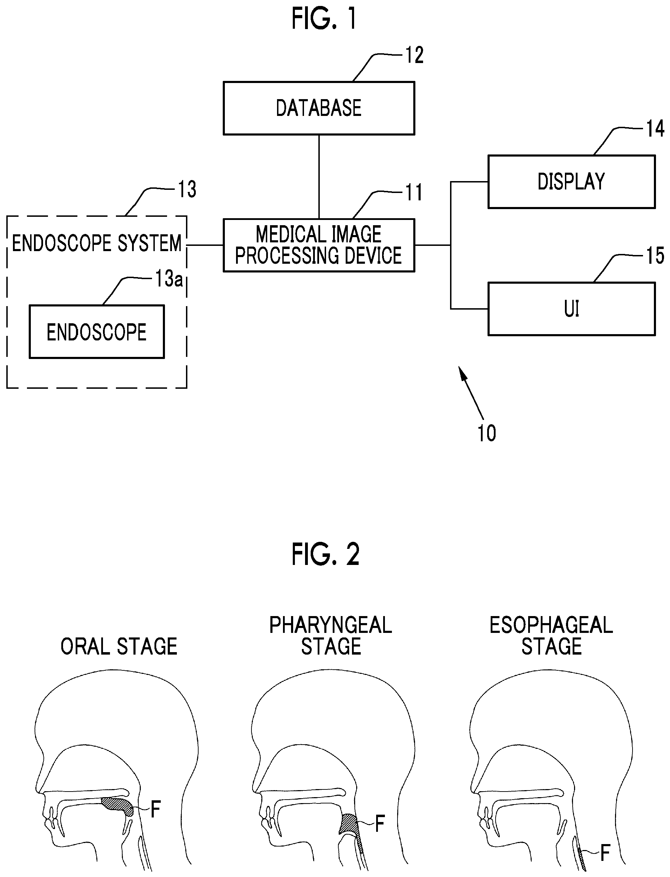

As illustrated in Fig. 1, a medical image processing system 10 includes a medical image processing device 11, a database 12, an endoscope system 13, a display 14, and a user interface 15. The medical image processing device 11 is electrically connected to the database 12, the endoscope system 13, the display 14, and the user interface 15. The database 12 is a device that can store acquired images and transmit and receive data to and from the medical image processing device 11, and may be a recording medium such as a universal serial bus (USB) or a hard disc drive (HDD). The medical image processing device 11 acquires an image captured in a swallowing examination from an endoscope 13a that constitutes the endoscope system 13. The user interface 15 is an input device that performs the input of settings to the medical image processing device 11 and the like and includes a keyboard, a mouse, and the like.

The endoscope 13a is a swallowing endoscope that is inserted from the nasal cavity of a patient and illuminates the vicinity of the pharynx with illumination light to observe and image swallowing. Since swallowing is a movement, a moving image is acquired in the swallowing examination. In addition, unless otherwise specified in the imaging of swallowing, white light is used as the illumination light, and a video signal of 60 frame images (60 fps (frames per second)) is acquired per second.

As illustrated in Fig. 2, swallowing is a series of movements in which food F in the mouth is chewed, swallowed, and transported to the esophagus. The progression of the swallowing includes an "oral stage" in which the food F in the mouth is transported from the tongue to the pharynx, a "pharyngeal stage" in which the food F is transported from the pharynx to the esophagus, and an "esophageal stage" in which the food F is transported from the esophagus to the stomach. In this embodiment, the "pharyngeal stage" of swallowing is set as a swallowing timing, and the pharynx is observed. A plurality of liquids or solids that are harmless to the human body even in a case in which they are swallowed and have different degrees of easiness to swallow are used as the food F. For example, milk, a colored aqueous solution, and pudding are used as the food F.

In the swallowing examination, an aspect in which the patient puts the food F into the mouth, swallows it in the pharynx, and transports it to the stomach through the esophagus is imaged. Even in a case in which the patient is not able to swallow the food F, the aspect is imaged. In the swallowing examination, it is preferable to continuously check a plurality of swallowing movements in succession instead of checking one swallowing movement at a time. For example, during the swallowing examination, the patient swallows the food F in the order of a colored aqueous solution, milk, his or her saliva, and pudding.

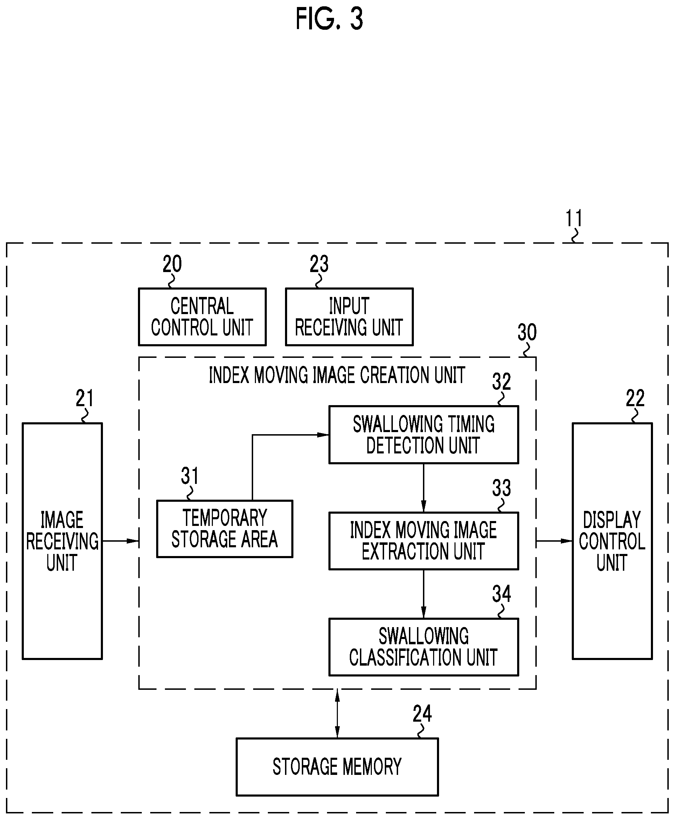

As illustrated in Fig. 3, in the medical image processing device 11, a program related to processing, such as image processing, is stored in a program memory (not illustrated). In the medical image processing device 11, a central control unit 20 configured by an imaging control processor operates the program in the program memory to implement the functions of an index moving image creation unit 30, an image receiving unit 21, a display control unit 22, an input receiving unit 23, and a storage memory 24. Further, with the implement of the function of the index moving image creation unit 30, the functions of a temporary storage area 31, a swallowing timing detection unit 32, an index moving image extraction unit 33, and a swallowing classification unit 34 are implemented.

The image receiving unit 21 receives a moving image file 41, on which the swallowing examination by the endoscope 13a has been recorded, and transmits the moving image file 41 to the index moving image creation unit 30. The index moving image creation unit 30 extracts an index moving image 42 from the moving image file 41 and transmits the index moving image 42 to the display control unit 22. The display control unit 22 performs control to display the index moving image 42 on the display 14. The input receiving unit 23 is connected to the user interface 15. The storage memory 24 can independently implement a storage function and is connected to the database 12 such that it can transmit and receive data. In addition, the moving image file 41 is received after the swallowing examination ends. However, a video signal may be processed in real time during the swallowing examination before the moving image file 41 is created.

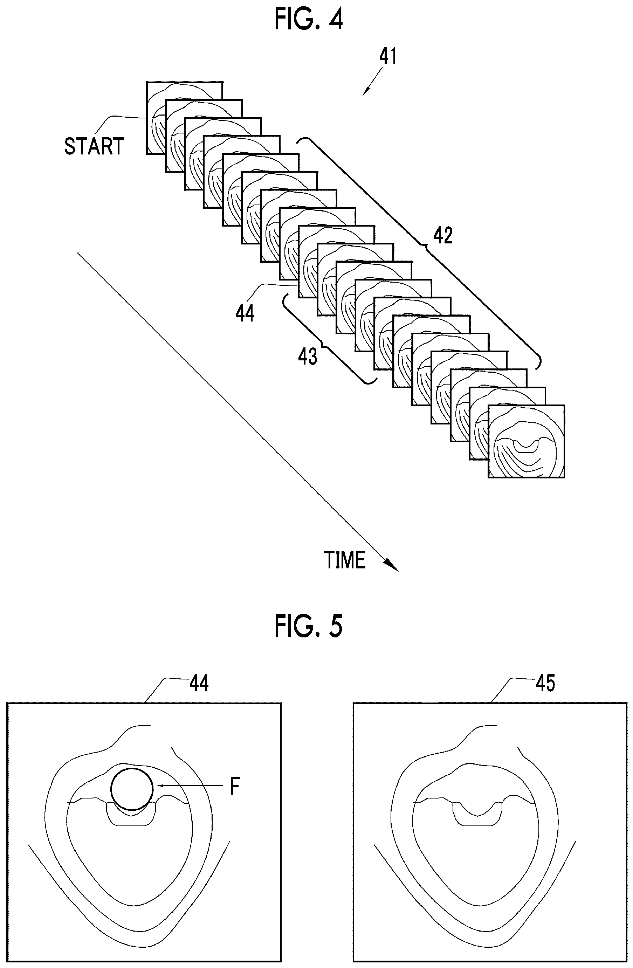

As illustrated in Fig. 4, the index moving image creation unit 30 excludes a scene in which swallowing is not performed from the moving image file 41 and extracts the index moving image 42 in order to efficiently observe swallowing. The index moving image 42 includes a swallowing frame image group 43 and frame image groups for predetermined period of time which are arranged before and after the swallowing frame image group 43 to be continuous with the swallowing frame image group 43. The swallowing frame image group 43 includes a plurality of continuous swallowing frame images 44, and the swallowing frame image 44 is a frame image that captures the swallowing timing. An arrow in Fig. 4 indicates the progress of time. In a case in which the moving image file 41 is played back, the frame images are played back and displayed along the direction of the arrow. A single frame image is one image, and an image group of continuous frame images is a moving image.

The moving image file 41 transmitted to the index moving image creation unit 30 is temporarily stored in the temporary storage area 31. The temporarily stored moving image file 41 is transmitted to the swallowing timing detection unit 32.

The swallowing timing detection unit 32 performs a swallowing detection process that analyzes the moving image file 41 with a tool only for machine learning using, for example, deep learning to determine whether or not to have the swallowing timing which is a movement in a case in which the food F passes through the pharynx. A portion corresponding to the swallowing timing is the swallowing frame image group 43 and is continuous frame images including the food F which are included in the moving image file 41. The moving image file 41 subjected to the swallowing detection process is transmitted to the index moving image extraction unit 33. Further, it is preferable that the moving image file 41, in which the swallowing timing has been detected, is stored in the storage memory 24 and is deleted from the temporary storage area 31.

Fig. 5 illustrates the swallowing frame image 44 which is a frame image that constitutes the moving image file 41 and includes the food F passing through the pharynx and a non-swallowing frame image 45 which is a frame image that constitutes the moving image file 41 and does not include the food F passing through the pharynx. The swallowing frame image 44 is a frame image in which the food F is recognized in the swallowing detection process and which is tagged with, for example, "swallowing timing detection". The non-swallowing frame image 45 is a frame image in which the swallowing timing is not recognized by machine learning, that is, the food F is not captured by deep learning in the swallowing detection process and which is not tagged with the "swallowing timing detection".

The index moving image extraction unit 33 extracts an index moving image including a frame tagged with the "swallowing timing detection" from the moving image file 41. Specifically, the index moving image extraction unit 33 extracts, as the index moving image 42, the swallowing frame image group 43 and frame image groups for a predetermined period which are continuous with the start and end of the swallowing frame image group 43 in the moving image file 41. For the extraction of the swallowing frame image group 43, the frame images tagged with the "swallowing timing detection" are extracted from the moving image file 41. Further, the predetermined period is a time of about 3 seconds or 5 seconds set in advance by the user and is preferably a period required to capture, for example, the movement of the pharynx before and after the passage of the food F.

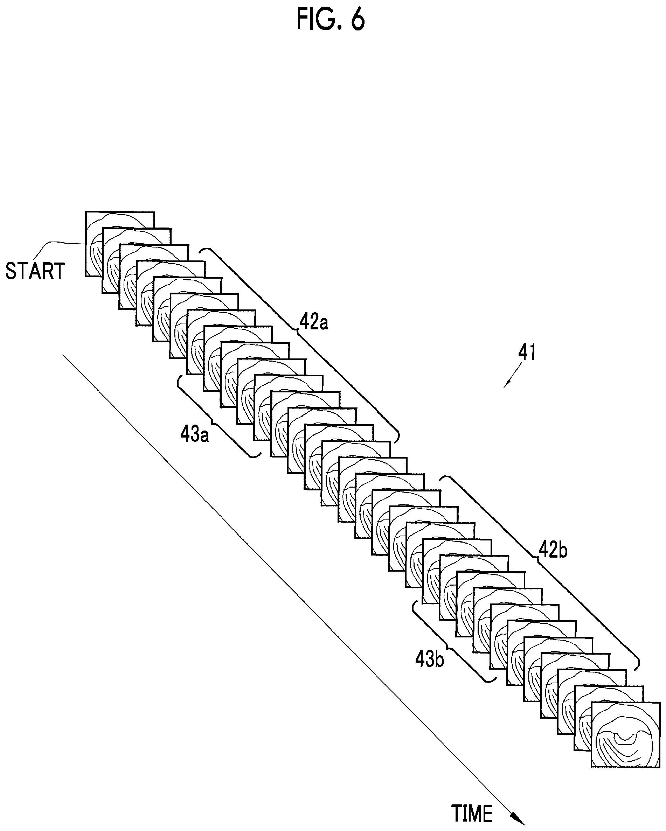

As illustrated in Fig. 6, in one moving image file 41, the number of times swallowing is observed is not limited to only one. In a case in which a plurality of swallowing timings are detected, a plurality of index moving images 42 are extracted from one moving image file. For example, in a case in which the swallowing timing detection unit 32 detects different swallowing timings 43a and 43b from one moving image file 41, the index moving image extraction unit 33 individually extracts an index moving image 42a having the swallowing timing 43a and an index moving image 42b having the swallowing timing 43b.

The swallowing classification unit 34 analyzes the index moving image 42, gives the types and the results of the swallowing examinations in the index moving image 42, or for example, index numbers. The classification result is the classification of the type of food F swallowed and includes, for example, the swallowing of saliva, the swallowing of milk, the swallowing of a colored aqueous solution, the swallowing of pudding, and whether the swallowing thereof is normal swallowing or aspiration. Score evaluation that gives a point according to the degree of aspiration may be used. These are automatically classified on the basis of data learned by machine learning. It is preferable to give information on the automatically classified type of swallowing examination food to the index moving image 42. The index numbers are numbers that are used by the user to search for the index moving images 42 stored in, for example, the storage memory 24 and are preferably alphanumeric character strings that do not overlap each other, have a small number of digits, and indicate, for example, the order in which the swallowing timing is detected and the type of swallowing examination food. In this case, the types of swallowing are saliva (sa), milk (mi), colored water (cw), pudding (pu), and unknown (un). In addition, the swallowing is classified into normal swallowing (S) or aspiration (A). The index moving image 42 after the classification is transmitted to the display control unit 22.

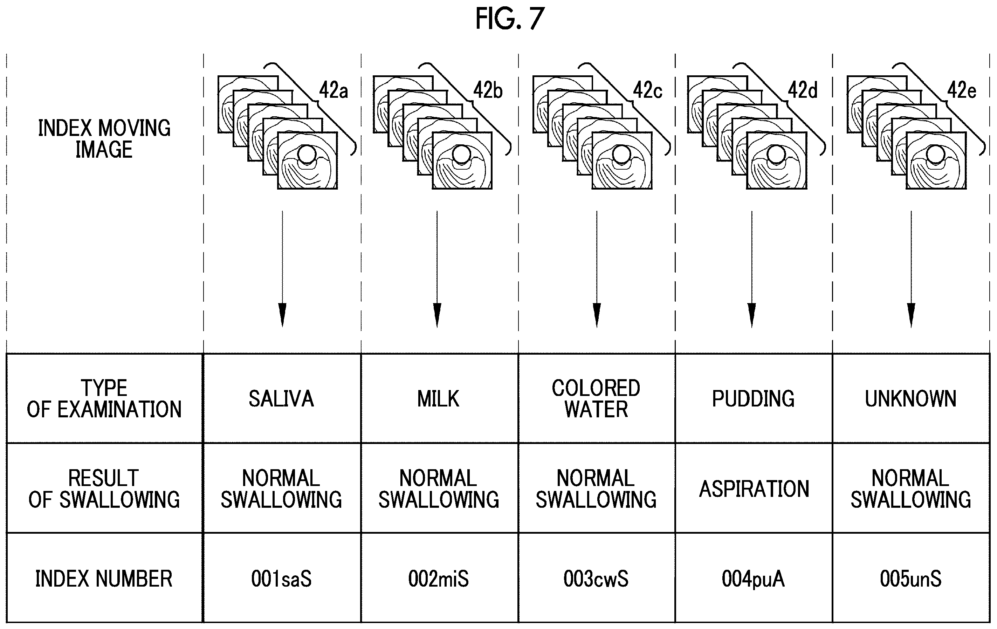

As illustrated in Fig. 7, for example, the swallowing classification unit 34 classifies the types of swallowing in index moving images 42a, 42b, 42c, 42d, and 42e extracted continuously, and the classification result is incorporated into the index number indicating the order in which the swallowing timing is detected. Assuming that the index moving images 42a to 42e are sequentially extracted in the order in which the swallowing timing is detected, 42a shows the normal swallowing of the saliva and is represented by "001saS", 42b shows the normal swallowing of the milk and is represented by "002miS", 42c shows the normal swallowing of the colored water and is represented by "003cwS", 42d shows the aspiration of the pudding and is represented by "004puA", and 42e shows the normal swallowing of the unknown swallowing examination food and is represented by "005unS".

For example, the display control unit 22 controls the switching of the screen displayed on the display 14. In a case in which the swallowing examination is performed to image the pharynx with the endoscope 13a, a real-time video observed by the user is displayed as an observation screen. In a case in which the moving image or the like acquired after the end of the imaging is displayed, the moving image is displayed as a playback screen.

The index moving image creation unit 30 automatically performs a process from the acquisition of the moving image file 41 to the transmission of the index moving image 42 to the display control unit 22. In a case in which the imaging by the endoscope 13a ends, the display of the display 14 is switched from the observation screen to the playback screen. The index moving image 42 extracted from the acquired moving image file 41 is displayed on the playback screen.

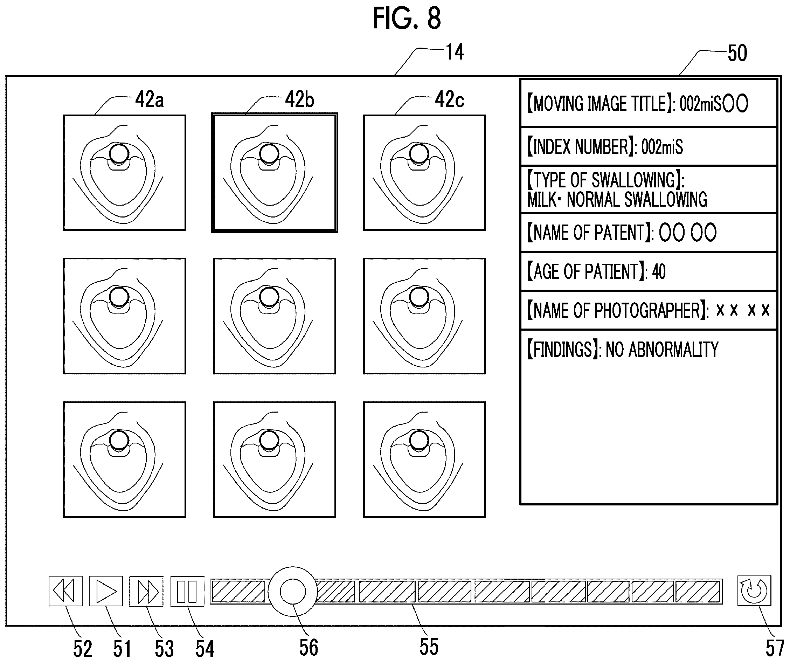

As illustrated in Fig. 8, each of the acquired index moving images 42 is displayed in a list on the playback screen of the display 14. The index moving images 42 displayed in the list are automatically played back such that the user can review the swallowing timing and compare the index moving images 42 without any operation. It is preferable that the index moving images are automatically played back one by one in the order in which they are captured. In a case in which the index moving images 42a, 42b, 42c ... are arranged in this order, they are played back in that order. It is preferable to display a moving image information display field 50, in which various kinds of information of the index moving image 42 that is being played back, on the playback screen at the same time. The playback screen comprises a play button 51, a fast rewind button 52, a fast forward button 53, a pause button 54, a seek bar 55, a slider 56, and a repeat play button 57 for the user to operate the playback of the moving image.

In the continuous playback of each index moving image 42, it is preferable to display the moving image that is being played back so as to be highlighted. For example, as illustrated in the index moving image 42b, the frame of the moving image is thickened to make it easier to see. The index moving image 42 is automatically played back at the time of automatic display such that the content displayed in the moving image information display field 50 can be checked and edited by the operation of the user. Information, such as the type of swallowing and the index number given by the swallowing classification unit 34, the name and age of the patient, the name of the photographer, the title of the moving image, and findings, is displayed in the moving image information display field 50. Further, the index number may be applied as the moving image title of the index moving image 42.

On the playback screen, the play button 51 can be pressed to repeatedly play back the index moving image 42 whose automatic playback has been ended, the fast rewind button 52 can be used to go back to the missed scene, the fast forward button 53 can be used to increase a moving image playback speed, and the pause button 54 can be used to stop playback at any time. The playback state of the moving image is represented by the position of the slider 56 on the seek bar 55, and the position of the slider 56 can be moved to freely change a playback point by the operation of the user such as dragging. In a case in which the repeat play button 57 is selected, the index moving image 42 whose playback has been ended is repeatedly played back. It is preferable that the seek bar 55 at the time of continuous playback displays the division of the playback time for each index moving image 42.

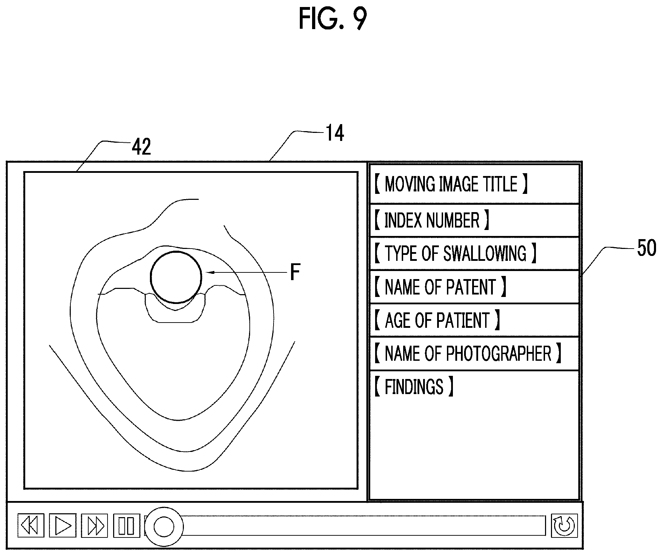

As illustrated in Fig. 9, the acquired index moving images 42 can be individually displayed on the display 14. The user can select any index moving image to be individually displayed from a plurality of index moving images 42 displayed in a list to switch the display mode to individual display. The index moving image 42 is automatically played back even in a case in which the display mode is switched to the individual display.

The user can check the information of the index moving image 42 displayed in the moving image information display field 50 and perform editing, such as addition and correction, on the information. For example, the addition of characteristics, such as findings obtained by playing back the index moving image 42 and the gender and age of the patient, and the editing of the automatically classified type of swallowing can be performed from the user interface 15 through the input receiving unit 23.

The user can edit the extraction range of the index moving image 42 in a case in which the information in the moving image information display field 50 is edited. For example, in a case in which the result of the review of the playback screen shows that the swallowing timing is erroneously detected or the extraction is insufficient from the index moving image 42, the index moving image 42 may be reacquired by re-extraction including manual extraction or re-examination with reference to the moving image file 41 stored in the temporary storage area 31.

The index moving image 42 in which various kinds of moving image information have been checked and edited is stored in the storage memory 24 or the database 12. It is preferable to delete the moving image file 41 which is an extraction source stored in the temporary storage area 31.

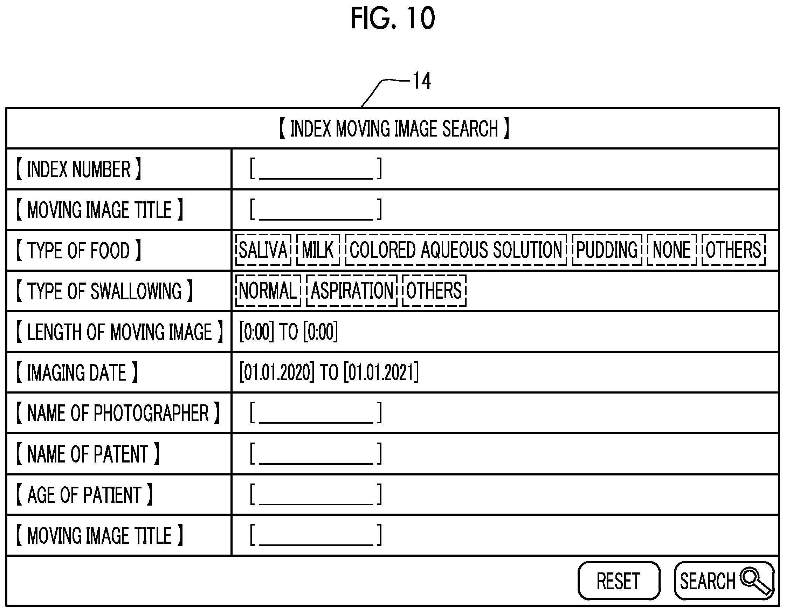

As illustrated in Fig. 10, in a case in which there are a large number of index moving images 42 to be played back on the playback screen, the user may select or input a keyword to search for a target index moving image 42. In that case, the display of the display 14 is switched to a search screen for searching. In addition to the index numbers, for example, the keywords of the type of swallowing food, the presence or absence of aspiration, patient information, and the content of findings are used for searching.

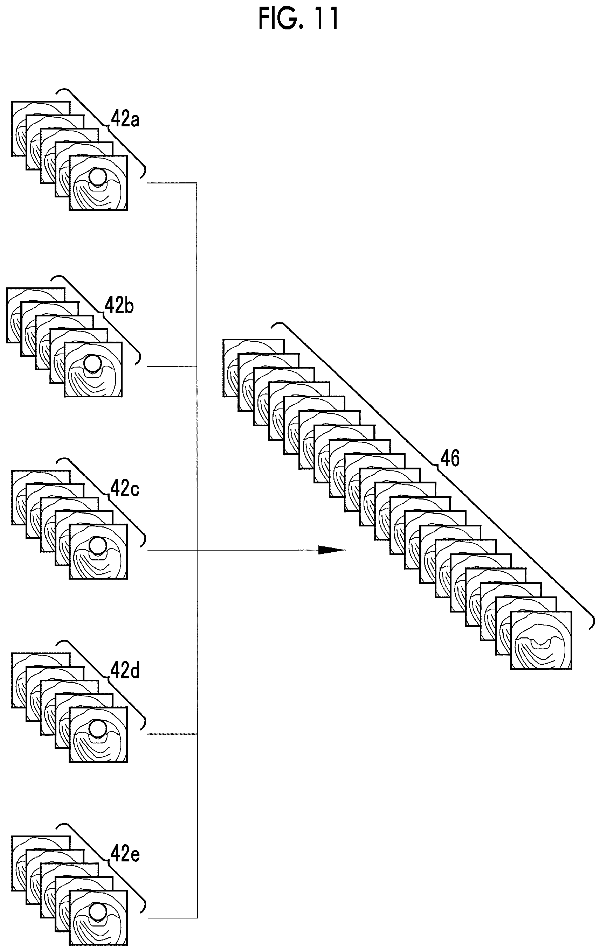

As illustrated in Fig. 11, after each index moving image 42 is stored, a plurality of any index moving images 42 may be combined to create one moving image such that a moving image that enables the user to continuously observe swallowing can be played back. For example, the index moving images 42a to 42e classified as in Fig. 7 may be combined to create a composite index moving image 46 such that swallowing is continuously checked in the order in which the images are captured. The index moving images 42 showing the same type of swallowing, normal swallowing, or aspiration may be combined to create the composite index moving image 46, in addition to the order. In addition, since the index number is given to each swallowing timing, an index number of the composite index moving image 46 is the sum of the index numbers of each swallowing timing.

The swallowing detection process will be described. In deep learning, a tool learns the characteristics of an image, which is an object to be detected, in advance. Then, the tool calculates the probability that the analyzed frame image will be the object to be detected, and a frame image having a probability equal to or greater than a threshold value is determined to be the object to be detected. The object to be detected is the pharynx that captures the food F, and the movement of the food F is tracked to detect the swallowing frame image 44. The swallowing frame image 44 is tagged. Further, in deep learning, it is necessary to learn information corresponding to the type of food F used for the swallowing examination.

The swallowing timing is the movement of the pharynx in a case in which the patient swallows the food F. However, deep learning for recognizing the food F is not needed to detect the swallowing timing, and the swallowing detection process may be performed according to, for example, the characteristics of the image showing the swallowing state or a change between the front and rear frame images. Specifically, the swallowing detection process may be performed by a swallowing detection algorithm using, for example, "a difference between the pixel values of the front and rear frame images", "the amount of blur of the frame image", and "the number of key points of the image". In addition, in any swallowing detection process, the frame image determined to shows the swallowing state is tagged with "swallowing timing detection".

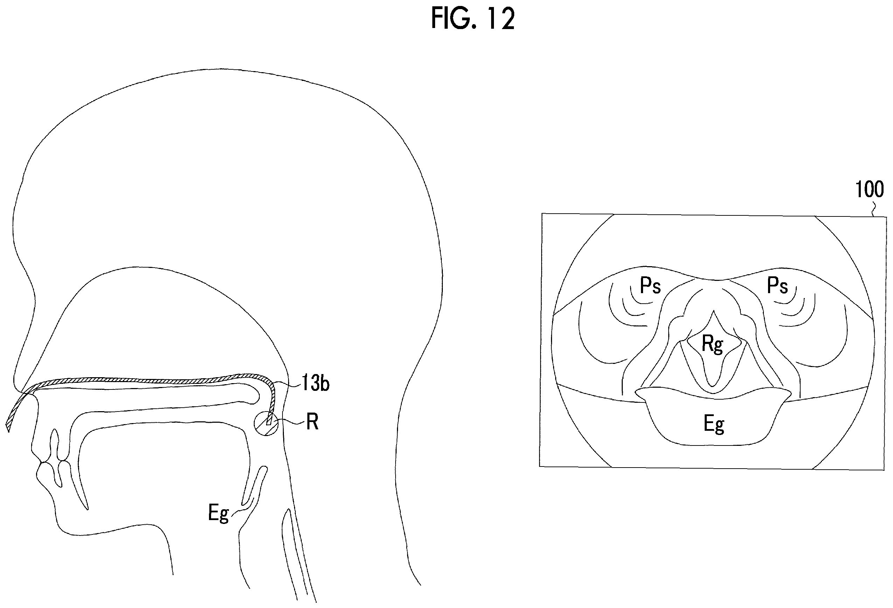

As illustrated in Fig. 12, a moving image is captured by locating the endoscope tip portion 13b, which is the tip of the endoscope 13a, at a position R. As illustrated in an example 100, it is preferable that a frame image constituting the moving image includes anatomical structures such as the epiglottis Eg, the rima glottidis Rg, and the left and right pyriform sinuses Ps. The rima glottidis Rg is a space between the left and right folds constituting the vocal cords.

In the detection algorithm using "the difference between the pixel values of the front and rear frame images", since the amount of movement of the object between the frame images is large in the images showing the swallowing state, the difference between the simple pixel values of the front and rear frame images is calculated. The swallowing timing detection unit 32 calculates the difference between the pixel values of two frame images that are continuous in time series. It is preferable to use AbsDiff installed in OpenCV (registered trademark) used for image analysis as a function for calculating the difference (simple difference value).

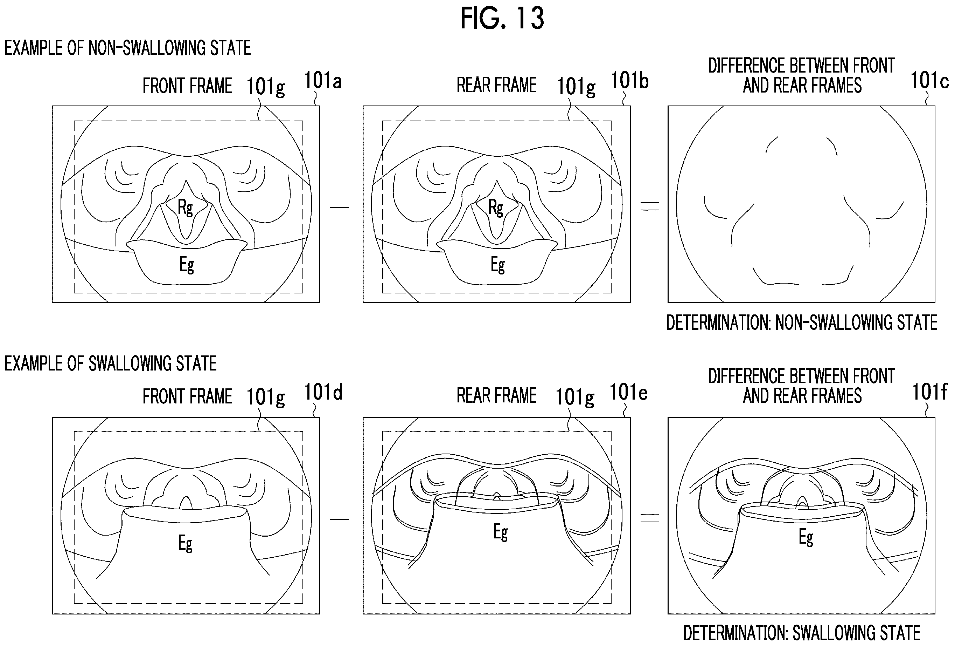

As illustrated in Fig. 13, a region (image processing target region 101g) which has at least 10 pixels or more from an image center of each frame image in the vertical direction and the horizontal direction is set as an image processing target, and a simple difference value, which is a difference between the pixel values of a front frame image (a front frame image 101a in an upper part of Fig. 13 and a front frame image 101d in a lower part of Fig. 13) and a rear frame image (a rear frame image 101b in the upper part of Fig. 13 and a rear frame image 101e in the lower part of Fig. 13) that are continuous in time series in the image processing target region 101g, is calculated. In a case in which the simple difference value is equal to or greater than a first threshold value, it is determined that the rear frame image 101b for which the simple difference value has been calculated shows the swallowing state. A first swallowing detection algorithm uses the fact that the simple difference value between the frame images is larger than that in the non-swallowing state since the object (particularly, the vicinity of the epiglottis Eg) moves violently with the swallowing movement. In addition, the pixel value which is a value of the first threshold value is preferably 1 to 255 and can be set to any value.

A specific example will be described in detail. The upper part of Fig. 13 is an example of the calculation of the simple difference value between two front and rear frame images (the front frame image 101a and the rear frame image 101b) in which the swallowing movement does not occur. In a state in which the swallowing movement does not occur, the epiglottis Eg is open, and it is possible to easily observe the rima glottidis Rg. In addition, the soft palate (not illustrated) which is the ceiling of the oral cavity or the epiglottis Eg hardly moves and is minutely moved by breathing. Therefore, the movement of the endoscope tip portion 13b is small, and there is little movement or blur in the entire image as illustrated in an example 101c of the difference in the upper part of Fig. 13. Therefore, the simple difference value between the front frame image 101a and the rear frame image 101b in the image processing target region 101g is less than the first threshold value, and it is determined that the rear frame image 101b shows the non-swallowing state. Further, in the example 101c of the difference, the difference to be calculated as the simple difference value is represented by a line.

The lower part of Fig. 13 illustrates an example of the calculation of the simple difference value between two front and rear frame images (the front frame image 101d and the rear frame image 101e) in which the swallowing movement is occurring. In a state in which the swallowing movement occurs, the movement of the soft palate or the closing movement of the rima glottidis Rg caused by the epiglottis Eg occurs. Therefore, the endoscope top portion 13b moves violently, and a large amount of blur occurs in the entire image. As illustrated in an example 101f of the difference in the lower part of Fig. 13, the difference between the frame images is large. In this case, the simple difference value between the front frame image 101d and the rear frame image 101e in the image processing target region 101g is equal to or greater than the first threshold value, and it is determined that the rear frame image 101e shows the swallowing state. In addition, as in the upper part, in the example 101f of the difference, the difference to be calculated as the simple difference value is represented by a line. Further, since the amount of blur between the frame images is large in the swallowing state, the number of lines indicating the difference in the example 101f of the difference in the lower part showing the swallowing state is larger than that in the example 101c of the difference in the non-swallowing state.

In the detection algorithm using "the amount of blur of the frame image", since the amount of blur of the object between the frame images is large in the images showing the swallowing state, the amount of edge indicating the amount of blur between the frame images is calculated. The swallowing timing detection unit 32 calculates the amount of edge related to the two frame images that are continuous in time series. It is preferable to use Variance Of Laplacian installed in OpenCV (registered trademark) used for image analysis as a function for calculating the amount of edge.

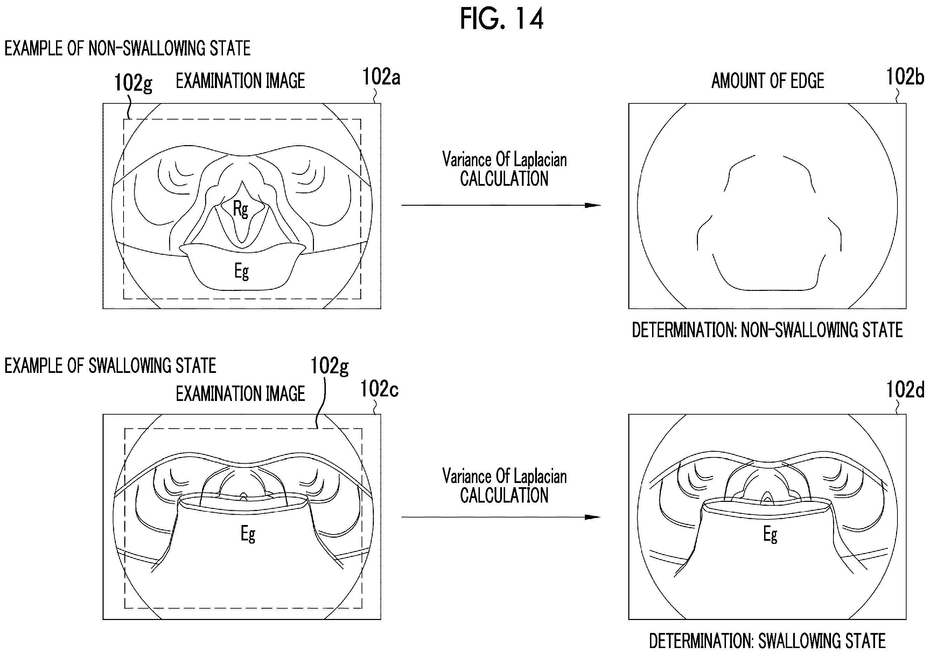

As illustrated in Fig. 14, a region (image processing target region 102g) which has at least 10 pixels or more from the image center of the frame image at least in the vertical direction and the horizontal direction in each frame image is set as the image processing target. The Variance Of Laplacian of the frame image (a frame image 102a in an upper part of Fig. 14 and a frame image 102c in a lower part of Fig. 14) is calculated, and the amount of edge (an example 102b of the amount of edge in the upper part of Fig. 14 and an example 102d of the amount of edge in the lower part of Fig. 14) is calculated. In a case in which the amount of edge is equal to or greater than a second threshold value, it is determined that the frame image for which the amount of edge has been calculated shows the swallowing state. A second swallowing detection algorithm uses the fact that, since the endoscope tip portion 13b moves violently during swallowing, the amount of edge increases as the amount of blur increases. In addition, the pixel value which is a value of the second threshold value is preferably 1 to 255 and can be set to any value.

A specific example will be explained in detail. The upper part of Fig. 14 illustrates an example of the calculation of the amount of edge of the frame image 102a in which the swallowing movement does not occur. In a state in which the swallowing movement does not occur, the soft palate or the epiglottis Eg hardly moves. Therefore, the endoscope tip portion 13b also hardly moves, and there is little movement or blur in the entire image as illustrated in the example 102b of the amount of edge in the upper part of Fig. 14. In this case, the amount of edge in the image processing target region 102g is less than the second threshold value, and it is determined that the frame image 102a shows the non-swallowing state. Further, in the example 102b of the amount of edge, a portion which is an object to be calculated as the amount of edge is represented by a line.

The lower part of Fig. 14 illustrates an example of the calculation of the amount of edge of the frame image 102c in which the swallowing movement is occurring. In a state in which the swallowing movement is performed, the soft palate or the epiglottis Eg moves, and the endoscope tip portion 13b also moves greatly, which causes blur in the entire image. Therefore, the amount of edge is large as illustrated in the example 102d of the amount of edge in the lower part of Fig. 14. In this case, the amount of edge in the image processing target region 102g is equal to or greater than the second threshold value, and it is determined that the frame image 102c shows the swallowing state. In addition, as in the upper part, in the example 102d of the amount of edge, a portion which is the object to be calculated as the amount of edge is represented by a line. Further, since the amount of blur is large during swallowing, the number of lines, for which the amount of edge is to be calculated, in the example 102d of the amount of edge in the lower part showing the swallowing state is larger than that in the example 102b in the non-swallowing state.

In the detection algorithm using the "number of key points", since the images showing the swallowing state have a large amount of blur of the object between the frame images, the edges of the frame images are unclear and the number of key points is reduced. The key point means a feature point which is a portion having a high probability of being a corner with a large amount of edge among the edges obtained by extracting lines representing the frame image. The swallowing timing detection unit 32 calculates the number of key points related to the frame image. It is preferable to use Count Key Points installed in OpenCV (registered trademark) used for image analysis as a function for calculating the number of key points.

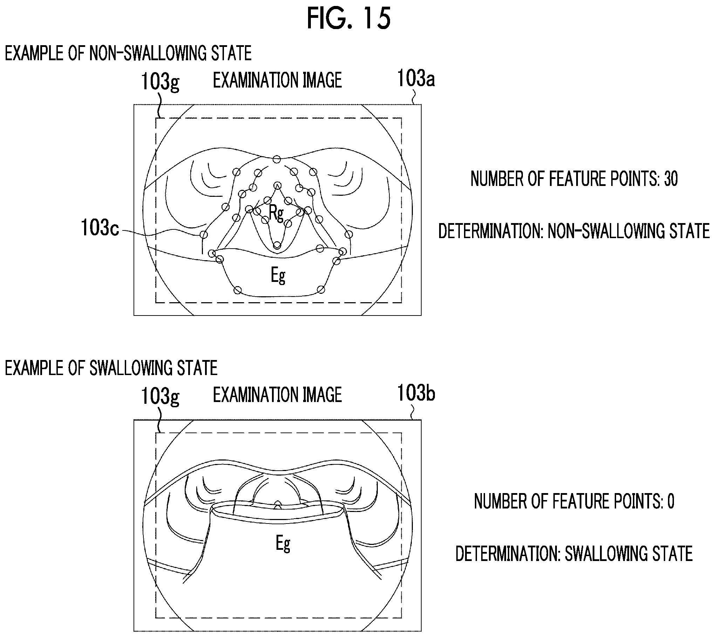

As illustrated in Fig. 15, a region (image processing target region 103g) that has at least 10 pixels or more from the image center of the frame image at least in the vertical direction and the horizontal direction in each frame image is set as the image processing target. As illustrated in Fig. 15, feature points 103c are extracted from the frame image (a frame image 103a in an upper part of Fig. 15 and a frame image 103b in a lower part of Fig. 15), and the number of feature points is calculated. In a case in which the number of feature points 103c is equal to or less than a third threshold value, it is determined that the frame image for which the feature points have been calculated is determined to show the swallowing state. A third swallowing detection algorithm uses the fact that, since the endoscope tip portion 13b moves violently during swallowing, the amount of blur is large. In addition, a value of the third threshold value is equal to or greater than 0 and can be set to any value. Further, in a case in which the number of feature points is equal to or less than the third threshold value, a swallowing determination value obtained by multiplying the pixel value by -1 may be calculated. In a case in which the swallowing determination value is less than a threshold value, it may be determined that the frame image shows the swallowing state. In a case in which the number of feature points is greater than the third threshold value, it is determined that the frame image shows the non-swallowing state.

In addition, Accelerated KAZE (AKAZE) installed in OpenCV (registered trademark) used for image analysis is used to extract the feature points. In the extraction of the feature points in this embodiment, it is preferable to recognize a portion (a portion recognized as a "corner") having a large amount of edge in the image.

A specific example will be explained in detail. The upper part of Fig. 15 illustrates an example of the calculation of the number of feature points 103c of the frame image 103a in which the swallowing movement does not occur. In a state in which the swallowing movement does not occur, the endoscope tip portion 13b hardly moves, and there is little movement or blur in the entire image. Therefore, as illustrated in the frame image 103a in the upper part of Fig. 15, the number of detected feature points 103c is large. In the example illustrated in Fig. 15, in a case in which the third threshold value is 5, the number of feature points 103c in the image processing target region 103g is 30 that is greater than the third threshold value. Therefore, it is determined that the frame image 103a shows the non-swallowing state.

The lower part of Fig. 15 illustrates an example of the calculation of the number of feature points 103c of the frame image 103b in which the swallowing movement is occurring. In a state in which the swallowing movement occurs, the endoscope tip portion 13b moves greatly. Therefore, blur occurs in the entire image, and it is difficult to detect the feature points 103c as illustrated in the frame image 103b in the lower part of Fig. 15. Assuming that the third threshold value is 5 in the example illustrated in Fig. 15, the number of feature points 103c in the image processing target region 103g is 0 that is equal to or less than the third threshold value. Therefore, it is determined that the frame image 103b shows the swallowing state.

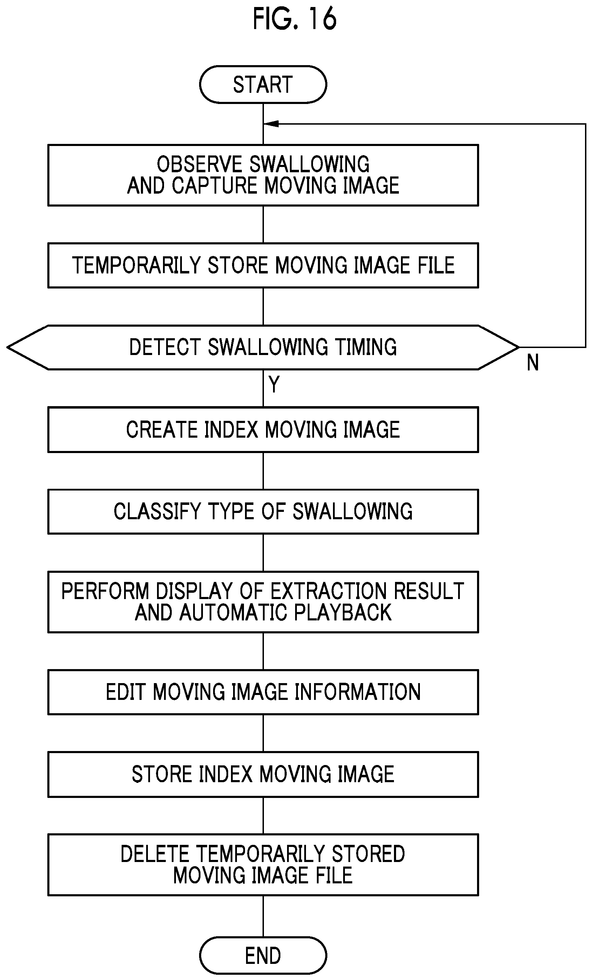

Fig. 16 is a flowchart illustrating a series of flow of automatic extraction of the index moving image 42 including the swallowing timing. The flow of extracting the index moving image 42 will be described below. The user acquires the moving image file 41 showing the aspect of the swallowing examination in the pharynx captured by the endoscope 13a. The moving image file 41 is transmitted to the index moving image creation unit 30. The moving image file 41 transmitted to the index moving image creation unit 30 is stored in the temporary storage area 31. Then, the swallowing timing detection unit 32 analyzes the moving image file 41 to specify the swallowing frame image group 43 including the frame images obtained by capturing the swallowing timing. The moving image file 41 in which the swallowing frame image group 43 has been specified is transmitted to the index moving image extraction unit 33. The swallowing frame image group 43 and the frame images for a predetermined period of time before and after the swallowing frame image group 43 are extracted as the index moving image 42. The index moving image 42 is transmitted to the swallowing classification unit 34 and is given the type of swallowing classified by machine learning or the index number. Then, the index moving image 42 is transmitted to the display control unit 22, is displayed in a list on the display 14, and is then automatically played back. After the automatic playback is performed and the index moving image 42 is reviewed, the classification result of each index moving image 42 or the like can be checked, and editing, such as addition or correction, can be performed. In a case in which the number of captured index moving images 42 displayed for the number of swallowing timings is insufficient due to, for example, an extraction failure or in a case in which image quality is poor, re-examination or manual extraction may be performed. In a case in which a sufficient number of index moving images 42 can be acquired, the moving image information is edited for the information displayed in the moving image information display field 50. The index moving image 42 whose moving image information has been edited is stored in the storage memory 24 in the medical image processing device or the database 12 of the connected device. Further, it is preferable to delete the moving image file 41 stored in the temporary storage area 31 in a case in which the moving image file 41 is not necessary.

### Second Embodiment

In the above-described embodiment, image analysis is performed on the acquired moving image file 41 to detect the swallowing timing. However, in this embodiment, the determination of whether or not the swallowing timing is present and classification are performed in addition to voice recognition at the time of swallowing. The extraction of the index moving image 42 according to this embodiment will be described below. In addition, the description of the same content as that in the above-described embodiment will not be repeated.

As the swallowing detection algorithm, voice is used to determine swallowing in the oral stage. The user interface 15 connected to the medical image processing device 11 includes a microphone (not illustrated) that acquires voice, and a voice waveform acquired from the microphone is input to the medical image processing device 11 from the input receiving unit 23 whenever the voice waveform is acquired. The acquisition of the voice is performed in operative association with the imaging of the pharynx, and the voice is transmitted to the index moving image creation unit 30 in a form in which it is attached to the moving image file 41. The voice waveform is associated as a voice signal with the moving image file 41, is stored in the temporary storage area 31 of the index moving image creation unit 30, and is then transmitted to the swallowing timing detection unit 32.

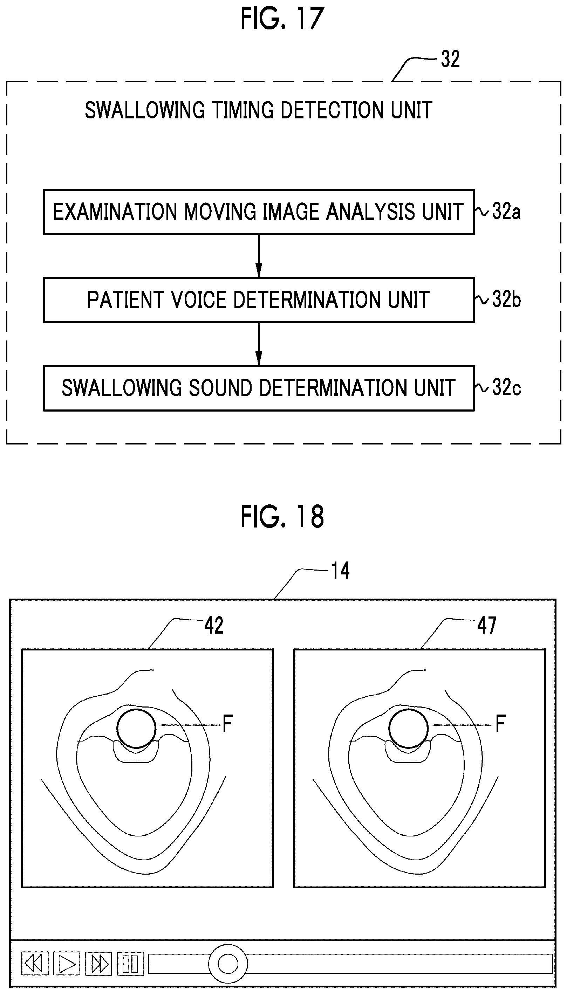

As illustrated in Fig. 17, in the second embodiment, the functions of an examination moving image analysis unit 32a, a patient voice determination unit 32b, and a swallowing sound determination unit 32c are implemented in the swallowing timing detection unit 32, and whether or not the swallowing timing is present is determined from image analysis and voice determination.

The examination moving image analysis unit 32a performs a process of performing image analysis on the moving image file 41 to detect swallowing, whose content is the same as that performed by the swallowing timing detection unit 32 in the first embodiment. Therefore, the moving image file 41 determined to have the food F recognized therein and to have the swallowing timing is transmitted to the patient voice determination unit 32b. In addition, it is preferable that the moving image file 41 in which the swallowing timing has been detected is stored in the storage memory 24. In this case, the moving image file 41 is deleted from the temporary storage area 31.

The patient voice determination unit 32b analyzes the voice signal that is attached to the moving image file 41 determined to have the swallowing timing to determine whether the voice is uttered from the patient or a person other than the patient. In a case in which the voice signal is determined to indicate the voice uttered from a person other than the patient, the voice signal is recorded together with the examination time. In a case in which the voice determined to be uttered from a person other than the patient is a specific voice (for example, a voice "examination start") uttered by a doctor or the like, the specific voice and the frame image of the moving image file 41 at the time when the specific voice is uttered may be associated with each other. In a case in which it is determined that the voice signal has the voice uttered from the patient at the swallowing timing, the voice signal is transmitted to the swallowing sound determination unit 32c. Further, the frame image of the moving image file 41 operatively associated with the determined voice may be tagged with a "patient voice" or a "non-patient voice".

The swallowing sound determination unit 32c determines whether the voice signal is a swallowing-related sound or a non-swallowing-related sound. Examples of the swallowing-related sound include a swallowing sound and epiglottis opening and closing sounds associated with swallowing. Examples of the non-swallowing-related sound include a coughing sound, a choking sound, a breathing sound, and a vocalized sound. In a case in which the voice signal is the swallowing-related sound, the frame image of the moving image file 41 operatively associated with the swallowing-related sound is tagged with the "swallowing-related sound". Similarly, the frame image of the moving image file 41 operatively associated with the non-swallowing-related sound is tagged with the "non-swallowing-related sound". The moving image file 41 is transmitted to the index moving image extraction unit 33 regardless of whether the swallowing-related sound is present in the voice signal. In addition, it is preferable to calculate the probability of the swallowing state to determine the swallowing-related sound.

In a case in which it is not possible to determine whether a swallowing reaction or a reaction other than swallowing occurs only using image analysis or in a case in which a reaction other than swallowing, such as the closing of the glottis or the opening and closing of the epiglottis due to coughing, occurs and the amount of movement of the glottis or the epiglottis is large even though accuracy is low, the swallowing timing detection unit 32 having the above-mentioned configuration can exclude these reactions other than swallowing using the voice signal, which makes it possible to improve the accuracy of determining the swallowing state or the non-swallowing state. Further, it is preferable that the index moving image extraction unit 33 extracts the moving image file 41, which has been determined to have the swallowing timing in the image analysis, but has not been tagged with the "swallowing-related sound" on the basis of only the "non-swallowing-related sound" by the swallowing sound determination unit 32c and then the swallowing classification unit 34 classifies whether or not the type of swallowing is aspiration.

The index moving image extraction unit 33 sets the frame images of the moving image file 41 tagged with the "swallowing-related sound" as the swallowing frame image group 43 at the swallowing timing. The index moving image extraction unit 33 extracts, as the index moving image 42, the swallowing frame image group 43 and the frame images for predetermined seconds which are continuous before and after the swallowing frame image group 43. In addition, for the moving image file 41 that has not been tagged with the "swallowing-related sound", the index moving image 42 is extracted only by the same image analysis as that in the first embodiment.

The swallowing classification unit 34 performs classification based voice analysis on the index moving image 42 in addition to the classification based on the image analysis. At the time when the swallowing-related sound and the non-swallowing-related sound are generated, the swallowing classification unit 34 classifies the types of swallowing into normal swallowing or abnormal swallowing (swallowing disorder) and gives the classification result to the index moving image 42. Specifically, the classification of the normal swallowing or the abnormal swallowing related to the swallowing-related sound and the non-swallowing-related sound is determined after the following are analyzed: the number of swallowing-related sounds and non-swallowing-related sounds; the nature and length of the swallowing sound; breathing sounds before and after swallowing; choke and cough after swallowing; and at what interval the swallowing-related sounds are uttered in a case in which the swallowing-related sound is uttered a plurality of times; and whether or not the epiglottis opening and closing sounds associated with swallowing are related to swallowing disorder. The classification result by the voice analysis can be combined with the classification result by the image analysis to obtain a more specific classification result or a classification result with high accuracy.

After the classification is performed by the swallowing classification unit 34, the index moving image 42 is displayed and then automatically played back on the display 14 through the display control unit 22. It is preferable that the index moving image 42 which is automatically played back is also played back in operative association with the swallowing-related sound. Further, it is preferable that, for example, information of whether or not swallowing is normal is automatically displayed in the information described in the moving image information display field 50.

### Third Embodiment

In each of the above-described embodiments, the medical image processing device 11 acquires the captured moving image file 41 from the endoscope system 13 and extracts the index moving image 42. However, in this embodiment, in addition to the extraction according to each of the above-described embodiments, the index moving image 42 is extracted from the moving image file 41 stored in the database 12. The review of the swallowing examination according to this embodiment will be described below. In addition, the description of the same content as that in the above-described embodiment will not be repeated.

Some swallowing examinations are performed a plurality of times at intervals in order to track a change in the condition of a disease. Therefore, it is desirable to compare the acquired results of the swallowing examination with the results of the swallowing examination performed in the past. The medical image processing device 11 receives the moving image file 41 obtained by capturing the swallowing examination in the past from the database 12 with the image receiving unit 21 and extracts the index moving image with the index moving image creation unit 30.

As illustrated in Fig. 18, the index moving image 42 acquired and extracted from the endoscope system 13 and a past index moving image 47 acquired and extracted from the database 12 are displayed side by side on the display 14 to compare the swallowing aspects of the food F.

In a case in which a specific moving image file 41 is acquired from the database 12, for example, it is preferable that the specific moving image file 41 is acquired by a search from a search screen using the type name of swallowing, the name of the patient, the imaging date, and the like in order to check whether or not the swallowing in the acquired index moving image 42 is normal.

The moving image acquired from the database 12 may be the moving image file 41 to be subjected to the extraction process, or the extracted past index moving image 47 may be directly acquired from the database 12 and then displayed on the display 14. Further, the index moving image 42 and the past index moving image 47 may be combined to obtain the composite index moving image 46 illustrated in Fig. 11. The composite index moving image 46 of the same swallowing type of the same patient on different examination dates is suitable for tracking a change in the condition of the disease.

In each of the above-described embodiments, the hardware structures of the processing units executing various processes, such as the central control unit 20, are the following various processors. The various processors include, for example, a central processing unit (CPU) which is a general-purpose processor that executes software (programs) to function as various processing units, a programmable logic device (PLD), such as a field programmable gate array (FPGA), that is a processor whose circuit configuration can be changed after manufacture, and a dedicated electric circuit that is a processor having a dedicated circuit configuration designed to perform various processes.

One processing unit may be configured by one of the various processors or a combination of two or more processors of the same type or different types (for example, a combination of a plurality of FPGAs and a combination of a CPU and an FPGA). Further, a plurality of processing units may be configured by one processor. A first example of the configuration in which a plurality of processing units are configured by one processor is an aspect in which one processor is configured by a combination of one or more CPUs and software and functions as a plurality of processing units. A representative example of this aspect is a client computer or a server computer. A second example of the configuration is an aspect in which a processor that implements the functions of the entire system including a plurality of processing units using one integrated circuit (IC) chip is used. A representative example of this aspect is a system-on-chip (SoC). As described above, various processing units are configured using one or more of the various processors as a hardware structure.

In addition, specifically, the hardware structure of the various processors is an electric circuit (circuitry) obtained by combining circuit elements such as semiconductor elements. Further, the hardware structure of the storage unit is a storage device such as a hard disc drive (HDD) or a solid state drive (SSD).

### Explanation of References

10: medical image processing system
11: medical image processing device
12: database
13: endoscope system
13a: endoscope
13b: endoscope tip portion
14: display
15: user interface
20: central control unit
21: image receiving unit
22: display control unit
23: input receiving unit
24: storage memory
30: index moving image creation unit
31: temporary storage area
32: swallowing timing detection unit
32a: examination moving image analysis unit
32b: patient voice determination unit
32c: swallowing sound determination unit
33: index moving image extraction unit
34: swallowing classification unit
41: moving image file
42: index moving image
42a: index moving image
42b: index moving image
42c: index moving image
42d: index moving image
42e: index moving image
43: swallowing frame image group
43a: swallowing frame image group
43b: swallowing frame image group
44: swallowing frame image
45: non-swallowing frame image
46: composite index moving image
47: past index moving image
50: moving image information display field
51: play button
52: fast rewind button
53: fast forward button
54: pause button
55: slider
56: seek bar
57: repeat play button
100: example of frame image in Fig. 12
101a: front frame image in upper part of Fig. 13
101b: rear frame image in upper part of Fig. 13
101c: example of difference in upper part of Fig. 13
101d: front frame image in lower part of Fig. 13
101e: rear frame image in lower part of Fig. 13
101f: example of difference in lower part of Fig. 13
101g: image processing target region in Fig. 13
102a: frame image in upper part of Fig. 14
102b: example of amount of edge in upper part of Fig. 14
102c: frame image in lower part of Fig. 14
102d: example of amount of edge in lower part of Fig. 14
102g: image processing target region in Fig. 14
103a: frame image in upper part of Fig. 15
103b: frame image in lower part of Fig. 15
103c: feature point
103g: image processing target region in Fig. 15
Eg: epiglottis
F: food
R: position
Rg: rima glottidis
Ps: pyriform sinus

## Claims

1. A medical image processing system comprising:
a processor (20) configured to:
receive a video signal on which a swallowing examination has been recorded by an endoscope;
analyze the video signal to determine whether or not a swallowing timing is present;
**characterised in that** the processor (20) is further configured to set a frame image at the swallowing timing as a swallowing frame image (44) tagged with swallowing timing detection, and extracts an index moving image (42) including the swallowing frame image from the video signal.

2. The medical image processing system according to claim 1,
wherein the index moving image includes a swallowing frame image group (43) including the swallowing frame image and a frame image group for a predetermined period which is continuous with the swallowing frame image group.

3. The medical image processing system according to claim 2,
wherein the frame image group for the predetermined period is non-swallowing frame images (45) which are arranged before a start of the swallowing frame image group and after an end of the swallowing frame image group and are not tagged with the swallowing timing detection.

4. The medical image processing system according to any one of claims 1 to 3,
wherein the video signal includes a frame image to be analyzed, and
the processor is configured to determine whether or not the swallowing timing is present using any one of calculation of an amount of blur of the frame image, calculation of a key point based on the frame image, or a difference between pixel values of the frame images.

5. The medical image processing system according to any one of claims 1 to 4,
wherein the processor is configured to analyze the index moving image to specify a type of the swallowing examination.

6. The medical image processing system according to any one of claims 1 to 4,
wherein the processor is configured to give an index number used to search for a moving image to the index moving image.

7. The medical image processing system according to any one of claims 1 to 4,
wherein the processor is configured to automatically play back the index moving image without any user operation in a case in which the index moving image is displayed on a screen.

8. The medical image processing system according to claim 7,
wherein the processor displays a plurality of the index moving images in a list on a display and automatically plays back the plurality of index moving images simultaneously or continuously.

9. The medical image processing system according to claim 1,
wherein the processor is configured to display at least one of a type of the swallowing examination or an index number in a case in which the index moving image is displayed on a screen.

10. The medical image processing system according to claim 1,
wherein the processor is configured to combine a plurality of the index moving images to create a composite index moving image in which the index moving images are capable of being continuously played back.

11. The medical image processing system according to claim 1,
wherein the processor is configured to further use voice recognition at the time of swallowing to determine whether or not the swallowing timing is present

12. A method for operating a medical image processing system including a processor, the method comprising:
a step of causing the processor to receive a video signal on which a swallowing examination has been recorded by an endoscope;
a step of causing the processor to analyze the video signal to determine whether or not a swallowing timing is present and to set a frame image at the swallowing timing as a swallowing frame image tagged with swallowing timing detection; and
a step of causing the processor to extract an index moving image including the swallowing frame image from the video signal.

## Patentansprüche

1. Verarbeitungssystem für medizinische Bilder, umfassend:
einen Prozessor (20), der so konfiguriert ist, dass er:
ein Videosignal, auf dem eine Schluckuntersuchung durch ein Endoskop aufgezeichnet wurde, empfängt;
das Videosignal analysiert, um zu bestimmen, ob ein Schluckzeitpunkt vorhanden ist oder nicht; **dadurch gekennzeichnet, dass** der Prozessor (20) ferner so konfiguriert ist, dass er
ein Einzelbild bei dem Schluckzeitpunkt als ein Schluckeinzelbild (44), das mit Schluckzeitpunkt-Detektion gekennzeichnet ist, einstellt, und ein Index-Bewegtbild (42), das das Schluckeinzelbild enthält, aus dem Videosignal extrahiert.

2. Verarbeitungssystem für medizinische Bilder nach Anspruch 1,
wobei das Index-Bewegtbild eine Schluckeinzelbild-Gruppe (43), die das Schluckeinzelbild enthält, und eine Einzelbildgruppe für einen vorbestimmten Zeitraum, der mit der Schluckeinzelbild-Gruppe kontinuierlich ist, enthält.

3. Verarbeitungssystem für medizinische Bilder nach Anspruch 2,
wobei die Einzelbildgruppe für den vorbestimmten Zeitraum Nicht-Schluckeinzelbilder (45) sind, die vor einem Start der Schluckeinzelbild-Gruppe und nach einem Ende der Schluckeinzelbild-Gruppe angeordnet sind und nicht mit der Schluckzeitpunkt-Detektion Schluckeinzelbild-Gruppe sind.

4. Verarbeitungssystem für medizinische Bilder V nach einem der Ansprüche 1 bis 3,
wobei das Videosignal ein Einzelbild, das zu analysieren ist, enthält, und
der Prozessor so konfiguriert ist, dass er unter Verwendung einer beliebigen von Berechnung einer Menge an Unschärfe des Einzelbildes, Berechnung eines Schlüsselpunkts auf der Grundlage des Einzelbildes oder einer Differenz zwischen Pixelwerten der Einzelbilder bestimmt, ob das Schluckzeitpunkt vorhanden ist oder nicht.

5. Verarbeitungssystem für medizinische Bilder nach einem der Ansprüche 1 bis 4,
wobei der Prozessor so konfiguriert ist, dass er das Index-Bewegtbild analysiert, um einen Typ der Schluckuntersuchung zu spezifizieren.

6. Verarbeitungssystem für medizinische Bilder nach einem der Ansprüche 1 bis 4,
wobei der Prozessor so konfiguriert ist, dass er dem Index-Bewegtbild eine Indexnummer, die verwendet wird, um nach einem Bewegtbild zu suchen, gibt.

7. Verarbeitungssystem für medizinische Bilder nach einem der Ansprüche 1 bis 4,
wobei der Prozessor so konfiguriert ist, dass er das Index-Bewegtbild automatisch ohne Benutzereingabe in einem Fall, in dem das Index-Bewegtbild auf einem Bildschirm angezeigt wird, wieder abspielt.

8. Verarbeitungssystem für medizinische Bilder nach Anspruch 7,
wobei der Prozessor mehrere der Index-Bewegtbilder in einer Liste auf einem Display anzeigt und die mehreren Index-Bewegtbilder automatisch gleichzeitig oder nacheinander abspielt.

9. Verarbeitungssystem für medizinische Bilder nach Anspruch 1,
wobei der Prozessor so konfiguriert ist, dass er mindestens eines von einem Typ der Schluckuntersuchung und einer Indexnummer in einem Fall, in dem das Index-Bewegtbild auf einem Bildschirm angezeigt wird, anzeigt.

10. Verarbeitungssystem für medizinische Bilder nach Anspruch 1,
wobei der Prozessor so konfiguriert ist, dass er mehrere der Index-Bewegtbilder kombiniert, um ein zusammengesetztes Index-Bewegtbild, bei dem die Index-Bewegtbilder in der Lage sind, kontinuierlich wiedergegeben zu werden, zu erstellen.

11. Verarbeitungssystem für medizinische Bilder nach Anspruch 1,
wobei der Prozessor so konfiguriert ist, dass er ferner Spracherkennung zu dem Zeitpunkt des Schluckens verwendet, um zu bestimmen, ob Schluckzeitpunkt vorhanden ist oder nicht.

12. Verfahren zum Betreiben eines Verarbeitungssystem für medizinische Bilder, das einen Prozessor enthält, wobei das Verfahren umfasst:
einen Schritt des Veranlassens, dass der Prozessor ein Videosignal, auf dem eine Schluckuntersuchung durch ein Endoskop aufgezeichnet wurde, empfängt;
einen Schritt des Veranlassens des Prozessors, das Videosignal zu analysieren, um zu bestimmen, ob ein Schluckzeitpunkt vorhanden ist oder nicht, und ein Einzelbild zu dem Schluckzeitpunkt als ein Schluckeinzelbild, das mit Schluckzeitpunkt-Detektion gekennzeichnet ist, einzustellen; und
einen Schritt des Veranlassens des Prozessors, ein Index-Bewegtbild, das das Schluckeinzelbild enthält, aus dem Videosignal zu extrahieren.

## Revendications

1. Système de traitement d'images médicales comprenant :
un processeur (20) configuré pour :
recevoir un signal vidéo sur lequel un examen de déglutition a été enregistré par un endoscope;
analyser le signal vidéo pour déterminer si un moment de déglutition est présent ou non ; **caractérisé en ce que** le processeur (20) est en outre configuré pour
définir une image de trame au moment de déglutition comme une image de trame de déglutition (44) marquée avec une détection de moment de déglutition, et extrait une image en mouvement indexée (42) incluant l'image de trame de déglutition à partir du signal vidéo.

2. Système de traitement d'images médicales selon la revendication 1,
dans lequel l'image en mouvement indexée inclut un groupe d'images de trame de déglutition (43) incluant l'image de trame de déglutition et un groupe d'images de trame pendant une période prédéterminée qui est contigu au groupe d'images de trame de déglutition.

3. Système de traitement d'images médicales selon la revendication 2,
dans lequel le groupe d'images de trame pendant la période prédéterminée est constitué d'images de trame sans déglutition (45) qui sont disposées avant un début du groupe d'images de trame de déglutition et après une fin du groupe d'images de trame de déglutition et qui ne sont pas marquées avec la détection de moment de déglutition.

4. Système de traitement d'images médicales selon l'une quelconque des revendications 1 à 3,
dans lequel le signal vidéo inclut une image de trame à analyser, et
le processeur est configuré pour déterminer si le moment de déglutition est présent ou non en utilisant l'un quelconque d'un calcul d'une quantité de flou de l'image de trame, d'un calcul d'un point clé sur la base de l'image de trame ou d'une différence entre des valeurs de pixel des images de trame.

5. Système de traitement d'images médicales selon l'une quelconque des revendications 1 à 4,
dans lequel le processeur est configuré pour analyser l'image en mouvement indexée pour spécifier un type de l'examen de déglutition.

6. Système de traitement d'images médicales selon l'une quelconque des revendications 1 à 4,
dans lequel le processeur est configuré pour attribuer un numéro d'index utilisé pour rechercher une image en mouvement à l'image en mouvement indexée.

7. Système de traitement d'images médicales selon l'une quelconque des revendications 1 à 4,
dans lequel le processeur est configuré pour lire automatiquement l'image en mouvement indexée sans aucune opération d'utilisateur dans un cas où l'image en mouvement indexée est affichée sur un écran.

8. Système de traitement d'images médicales selon la revendication 7,
dans lequel le processeur affiche une pluralité des images en mouvement indexées sous forme de liste sur un affichage et lit automatiquement la pluralité d'images en mouvement indexées simultanément ou en continu.

9. Système de traitement d'images médicales selon la revendication 1,
dans lequel le processeur est configuré pour afficher au moins l'un d'un type de l'examen de déglutition ou d'un numéro d'index dans un cas où l'image en mouvement indexée est affichée sur un écran.

10. Système de traitement d'images médicales selon la revendication 1,
dans lequel le processeur est configuré pour combiner une pluralité d'images en mouvement indexées pour créer une image en mouvement indexée composite dans laquelle les images en mouvement indexées peuvent être lues en continu.

11. Système de traitement d'images médicales selon la revendication 1,
dans lequel le processeur est configuré pour utiliser en outre la reconnaissance vocale au moment de la déglutition pour déterminer si le moment de déglutition est présent ou non.

12. Procédé de fonctionnement d'un système de traitement d'images médicales incluant un processeur, le procédé comprenant :
une étape consistant à amener le processeur à recevoir un signal vidéo sur lequel un examen de déglutition a été enregistré par un endoscope ;
une étape consistant à amener le processeur à analyser le signal vidéo pour déterminer si un moment de déglutition est présent ou non et à définir une image de trame au moment de déglutition comme une image de trame de déglutition marquée avec une détection de moment de déglutition ; et
une étape consistant à amener le processeur à extraire une image en mouvement indexée incluant l'image de trame de déglutition à partir du signal vidéo.
